# EUROPÄISCHE PATENTANMELDUNG

(11) **EP 1 295 626 A2**
(43) Veröffentlichungstag der Anmeldung: **26.03.2003**
(21) Anmeldenummer: 02021323.7
(22) Anmeldetag: 20.09.2002
(51) Int. Cl.: A61N 2/02

(54) **In einem Gerät Magnetspule zur pulsierenden Signaltherapie**

(30) Priorität: 20.09.2001 DE 10146428
(71) Anmelder: Markoll, Richard, Dr., 81371 München (DE)
(72) Erfinder: Markoll, Richard, Dr., 81371 München (DE)
(74) Vertreter: HOFFMANN - EITLE

(57) **Zusammenfassung**

Eine Magnetspule umfasst einen Kern (14) mit einer Längsachse und um den Kern gewickelte Spulenwindungen (12), wobei der Kern (14) durch gewickeltes, dünnes Blech gebildet ist. Solch eine Magnetspule (10) eignet sich insbesondere zur Anwendung in einem Gerät für die pulsierende Signaltherapie.

## Beschreibung

### Gebiet der Erfindung

Die Erfindung betrifft eine Magnetspule, die ein pulsierendes Signalfeld erzeugt, das beispielsweise zum Einsatz bei der pulsierenden Signaltherapie zur Behandlung akuter und chronischer Beschwerden am menschlichen Körper eingesetzt werden kann. Ferner betrifft die Erfindung die Verwendung einer Magnetspule in einem Gerät zur pulsierenden Signaltherapie.

Geräte zur pulsierenden Signaltherapie werden eingesetzt, um chronische und akute Leiden, etwa Gelenksbeschwerden aber auch andere Beschwerden, etwa Tinnitus, zu behandeln, indem sie stark schwankenden, pulsierenden Feldern mit einem vorgegebenen zeitlich variierenden Feld, einer bestimmten Feldstärke und einer vorgegebenen Frequenz des Feldes ausgesetzt werden. Beispielsweise hat sich zur Behandlung von Gelenksbeschwerden bzw. Beschwerden des musculo-skeletalen Systems herausgestellt, dass magnetische Felder mit einer Feldstärke von unter 20 Gauss, einer Frequenz von 1 bis 30 Hz und einer näherungsweise Rechtecksform im zeitlichen Verlauf äußerst wirkungsvoll sind.

### Stand der Technik

Die eingesetzten Spulen werden je nach Art des Einsatzes hinsichtlich der erzeugbaren Feldstärke gewählt und können beispielsweise zylindrische Spulen mit einer verhältnismäßig kurzen Spulenlänge sein, so dass sich das zu behandelnde Körperorgan in diese Zylinderspulen ohne Kern in der äußeren Gestalt eines Toroids einführen lässt und dort dem gerichteten Magnetfeld ausgesetzt wird. Diese Spulen können als Träger der Wicklung auch aus mehrere Spulensegmente umfassen, auf die eine entsprechende Wicklung (ringförmig) aufgebracht wird.

Bei der Verwendung dieser zylinderartigen Spulen wird das zu behandelnde Köperorgan, an dem die Beschwerden auftreten bzw. behandelt werden sollen, in die Spulenöffnung eingesetzt und das Organ bzw. die zu behandelnde Stelle wird zusammen mit ihrer unmittelbaren Umgebung dem pulsierenden Feld ausgesetzt.

Häufig ist es jedoch nicht wünschenswert bzw. möglich, das gesamte Körperorgan in ein zylindrisch gestaltetes Gehäuse, etwa in der Form eines Toroids, einzuführen.

Daher ist es ebenfalls bekannt, mehrere zylindrische Spulen mit Eisenkern so anzuordnen, dass ihre Hauptvektoren, also die Hauptfeldlinien, so gerichtet sind, dass sie an einem bestimmten Behandlungspunkt mit einer bestimmten Feldstärke zusammentreffen bzw. in ein bestimmtes Behandlungsgebiet treffen. Die dazu verwendeten Spulen sind gewöhnlich kurze zylindrische Spulen, etwa mit einer Spulenlänge von 2 bis 3 cm, um die Wicklungen geführt sind. Der Spulenkern ist dabei ein massiver Eisenkern, in dem ggf. eine Gewinde als Sack- oder Durchgangsloch vorgesehen sein kann, um die Spule an einer entsprechenden Vorrichtung zu befestigen.

### Darstellung der Erfindung

Es ist eine Aufgabe der Erfindung, eine Spule vorzusehen, die magnetische Felder mit äußerst präziser Ausrichtung erzeugt. Zusätzlich ist es eine Aufgabe der vorliegenden Erfindung, ein Gerät für die pulsierende Signaltherapie vorzusehen, bei dem ein sehr genau ausgerichtetes magnetisches Feld erzeugt wird.

Diese Aufgabe wird mit einer Magnetspule mit Merkmalen des Anspruchs 1 und der Verwendung einer Magnetspule gemäß Anspruch 5 erreicht.

Der Erfindung liegt der Gedanke zugrunde, den bislang in Spulen häufig vorgesehenen massiven Kern, meist einen Eisenkern, durch einen Kern zu ersetzen, der aus gewickeltem, dünnem Material, wie Blech, Cu oder anderes Material, das z. B. aus dem Einsatz für Federn bekannt ist, zu ersetzen. Die Wicklungen des Kerns können dabei beispielsweise durch die umgebende Spulenwicklung an ihrem Platz fixiert werden. Alternativ ist es auch möglich, die Wicklungen des Kerns durch Kunstharz zu fixieren, in das der Kern zumindest teilweise eingebettet ist. Selbstverständlich können auch andere Mittel Einsatz finden, um die federartig gewickelten Blechstreifen zu fixieren, etwa Klemmen oder Schrauben oder ähnliches.

Durch die Verwendung eines solchen gewickelten Kerns hat sich überraschenderweise herausgestellt, dass die Genauigkeit der Ausrichtung des magnetischen Felds, insbesondere des Hauptvektors des magnetischen Felds, der zur Behandlung im Rahmen der pulsierenden Signaltherapie auf einem bestimmten Behandlungsort gerichtet sein soll, in hohem Maß verbessert werden kann. Ebenfalls erhöht sich die Ausstrahlungslänge bei gleicher Stromzufuhr und Spulengröße um ca. 50%.

Vorteilhafte Ausführungsformen sind durch die übrigen Ansprüche gekennzeichnet.

Vorteilhafterweise ist die Magnetspule mit einem im Wesentlichen kreiszylindrischen Kern versehen. Dies ermöglicht eine kompakte Anordnung, insbesondere eine kompakte Wicklung der Spulenwindungen um den Kern. Zudem ist keine zusätzliche Verformung des gewickelten Kerns nach dem Wickeln notwendig.

Bevorzugterweise hat der Kern wie auch die gesamte Magnetspule eine verhältnismäßig kurze Spulenlänge, da dies zum Einbau in Geräte zur pulsierenden Signaltherapie vorteilhaft ist.

Bevorzugterweise werden die Spulenwindungen aus Kupferdraht als einem besonders leitfähigen und bewährten Medium gebildet. Nach einer vorteilhaften Ausführungsform wird der Kern aus Eisenblech gebildet. Auch hier werden die benötigten magnetischen Charakteristika einfach und sicher erfüllt, wobei zusätzlich die gute Verformbarkeit des Eisenblechs eine eng anliegende und sichere Wicklung ermöglicht.

Bei der Anordnung einer Spule in einem Gerät für die pulsierende Signaltherapie wird die Spule mit dem gewickelten Kern vorteilhafterweise so angeordnet, dass die Achse des Kerns bzw. der gesamten Spule in der Einsatzposition des Geräts auf den Anwendungspunkt der Signaltherapie, beispielsweise den locus cirillius, gerichtet ist. Es hat sich überdies vorteilhaft erwiesen, die Spulen so anzuordnen, dass sie etwa 8 bis 10 cm vom zu behandelnden Ort beabstandet sind. Durch den Einsatz der gewickelten Kerne sowie eine derartige Anordnung der Spulen kann eine sehr zielgerichtete Behandlung mit dem Gerät zur pulsierenden Signaltherapie durchgeführt werden, wobei das zu behandelnde Körperorgan bequem in dem Gerät zur pulsierenden Signaltherapie Platz findet.

Vorteilhafterweise sind mehrere Spulen vorgesehen, so dass ein Einwirken des Felds zur pulsierenden Signaltherapie auf das Körperorgan von mehreren Seiten möglich ist, wobei in diesem Fall nach einer bevorzugten Ausführungsform die Spulen und das zu behandelnde Organ so angeordnet sind, dass sie im Wesentlichen auf einem oder mehreren fiktiven Kreisen liegen, die ihren Mittelpunkt im Anwendungspunkt der Signaltherapie haben, wobei sich weiter bevorzugt die Verlängerung der Achsen der Spulen im Mittelpunkt dieses Kreises, also im Anwendungspunkt für die pulsierende Signaltherapie, schneiden. Dabei ist es auch möglich, mehrere Anordnungskreise mit unterschiedlichen Radien vorzusehen und/oder die Kreisringe in Ausrichtung der fiktiven Kreise in versetzten Ebenen anzuordnen. Dadurch kann einerseits eine punktgenaue Therapie durchgeführt werden, ohne die umgebenden Körperteile oder andere umgebende Einrichtungen unnötig pulsierenden Feldern auszusetzen. Insbesondere bei Einwirkung ungewollterweise auf umliegendes Gewebe oder andere Körperorgane könnte dies zu einer unerwünschten biomagnetischen Anregung führen.

Die Spulen sind vorzugsweise so in ihrer Stärke gewählt und positioniert, dass sie bei dem genannten Abstand von ca. 8 bis 10 cm vom Behandlungsort ein Magnetfeld am Behandlungsort erzeugen, dass unter 20 Gauss liegt, wobei sich das Magnetfeld durch Überlagerung der Felder der einzelnen Spulen, wenn mehrere vorgesehen werden, bildet. Die Spulen werden so betrieben, dass sie mit einer Frequenz von ca. 1 bis 30 Hz gepulst sind und durch Rechteckimpulse angetrieben werden.

Neben der genauen Ausrichtung des Felds haben die erfindungsgemäßen Spulen zusätzlich den Vorteil, dass die Wärmeentwicklung an den Spulen und damit am Gehäuse eines Geräts zur pulsierenden Signaltherapie innerhalb tolerierbarer Werte bleibt.

### Kurze Beschreibung der Zeichnungen

Nachfolgend wird die Erfindung rein beispielhaft anhand der beigefügten Figuren beschrieben, in denen:
Figur 1 eine schematische Ansicht einer erfindungsgemäßen Spule ist;
Figur 2 eine weitere perspektivische Ansicht durch die erfindungsgemäße Spule ist;
Figur 3 eine Querschnittsansicht in schematischer Form durch die Signalerzeugungseinheit eines Geräts zur pulsierenden Signaltherapie ist.

### Wege zur Ausführung der Erfindung

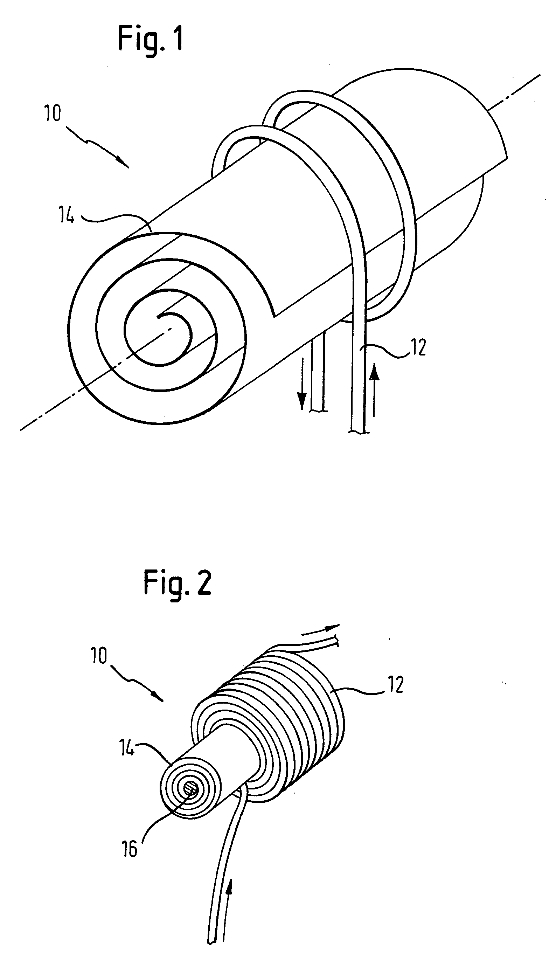

Figur 1 zeigt schematisch eine erfindungsgemäße Magnetspule 10. Die Spule umfasst Spulenwindungen 12, die z. B. aus Kupferdraht gefertigt sind, und die in typischer Weise für eine Zylinderspule um einen Kern 14 gewickelt sind. Die Spulenwindungen sind in bekannterweise an einen Stromkreis angeschlossen, so dass durch sie der Erregerstrom zum Erzeugen eines Magnetfelds geführt wird. In Figur 1 sind in der schematischen Darstellung nur wenige Spulenwindungen mit jeweils gleichem Durchmesser dargestellt. Selbstverständlich ist es auch möglich, und in der Praxis häufig angewandt, eine Vielzahl von Spulenwindungen vorzusehen und ggf. auch Spulenwindungen übereinander zu wickeln, so dass Spulenwindungen unterschiedlicher Durchmesser übereinander zu liegen kommen.

Wie es in Figur 1 gezeigt ist, erstreckt sich die Spulenwindung 12 gewöhnlicherweise nicht über die gesamte Länge des Kerns 14. Die Länge der Spulenwicklungen ist jedoch je nach den Anforderungen frei wählbar, so dass der Kern auf einer Seite der Spulenwicklung, gesehen in Längsrichtung, auf beiden Seiten der Spulenwicklung oder keiner Seite der Spulenwicklung vorstehen kann.

Der in Figur 1 dargestellte Kern 14 ist durch Wickeln eines dünnen, blechartigen Materials um die in Figur 1 schematisch dargestellte Kernachse gebildet. Gewöhnlicherweise entsteht dabei näherungsweise ein Kreiszylinder. Selbstverständlich ist es jedoch auch prinzipiell möglich, sollte sich dies als erforderlich weisen, nach dem Wickeln des blechartigen Materials diesem noch einem Verformungsprozess zu unterwerfen, so dass der Kern 14 als Zylinder mit beispielsweise einem elliptischen, rechteckigen oder anderem Querschnitt geformt wird. In der in Figur 1 dargestellten Ausführungsform ist die Kernachse gleichzeitig die Spulenachse der Magnetspule 10, also auch der Windungen 12. Dies ist jedoch ebenfalls nicht zwingend erforderlich.

Figur 2 zeigt eine weitere Ausführungsform der erfindungsgemäßen Spule. Insbesondere ist aus Figur 2 deutlich zu erkennen, dass der Kern 14 einseitig in Längsrichtung über die Spulenwindungen 12 vorsteht und die Spulenwindungen in mehreren Lagen ausgebildet sind. Zusätzlich ist in der in Figur 2 dargestellten Ausführungsform eine mit Gewinde versehene Bohrung 16 in den Kern eingebracht, die dazu dient, den Kern an einem Gehäuse zu fixieren. Diese Bohrung 16 kann als Sackloch ausgebildet sein oder aber als Durchgangsloch. Ebenso ist es möglich zusätzlich oder stattdessen andere Mittel zur Befestigung neben Gewindebohrungen, die mit entsprechenden Schrauben in Wechselwirkung kommen, einzusetzen.

Zum Einbringen der Gewindebohrung 16 in den Kern 14 ist festzuhalten, dass dies beispielsweise dadurch möglich ist, dass eine entsprechend feste, eng anliegende Wicklung des blechartigen Materials zum Bilden des gewickelten Kerns vorgenommen wird. In diese Wicklungen kann dann die Gewindebohrung eingebracht werden. Überdies können z. B. die gewickelten Schichten des Kerns 14 durch Kunstharz oder ähnliches, beispielsweise Klebemittel, fixiert werden, so dass das Einbringen von Haltemitteln oder eines Gewindebohrlochs ebenfalls möglich ist.

Die Pfeile in Figur 1 und 2 geben schematisch die Richtung des Stromflusses durch die Spule 12 an. Die Richtung des Stammflusses bezüglich der Wicklungsrichtung des Kerns ist jedoch ebenfalls ein frei wählbarer Parameter. Insbesondere kann die Richtung der Wicklung der Spulenwindungen 12 im Bezug auf die Richtung der Wicklung des Kerns 14 freigewählt werden, was bedeutet, dass die Wicklung des Kerns 14 und die Spulenwicklungen 12 entweder gleich oder entgegengesetzt gerichtet sein können.

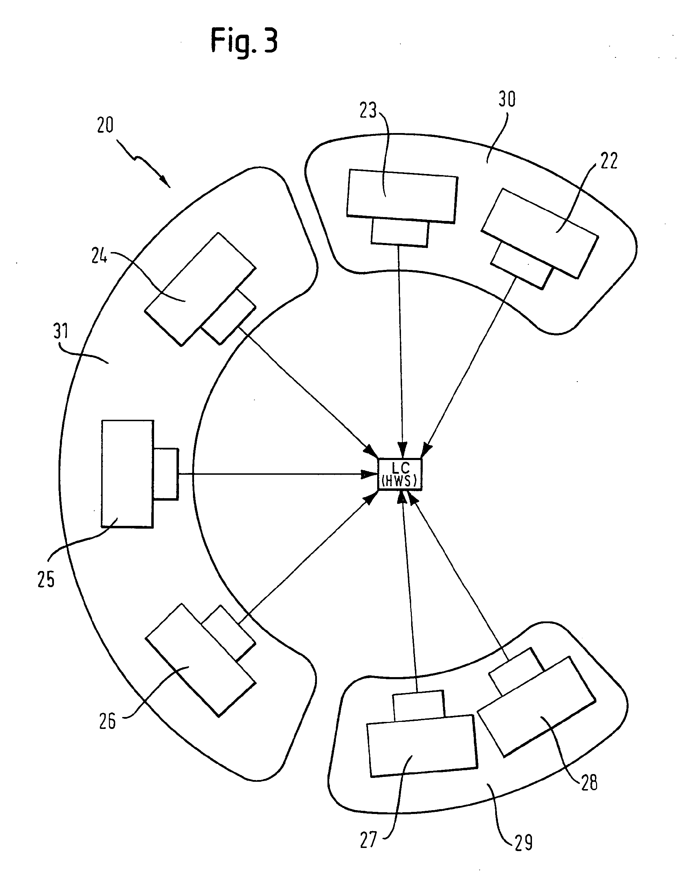

Figur 3 zeigt schließlich schematisch einen Querschnitt durch ein Signalerzeugungselement 20 für ein Gerät zur pulsierenden Signaltherapie (nicht dargestellt).

Das dargestellte Signalerzeugungselement 20 ist im Wesentlichen halbkreisbogenförmig, so dass beispielsweise, wie in der dargestellten Ausführungsform angedeutet, der Kopf eines Patienten darin ruhen kann oder das Signalerzeugungselement über dem Kopf gehalten werden kann. Mit "LC" ist in Figur 3 der zu behandelnde Ort, im Beispiel der locus cirillius zur Tinnitus-Behandlung, dargestellt. Das halbkreisbogenförmige Signalerzeugungselement eines Geräts zur pulsierenden Signaltherapie kann überdies mit Polstern oder ähnlichem versehen sein, insbesondere wenn der Kopf des Patienten oder ein anderes Körperteil darauf aufliegen soll.

In der In Figur 3 dargestellten Ausführungsform sind 7 Spuleneinrichtungen 22, 23, 24, 25, 26, 27, 28 vorgesehen. Jede der Spuleneinrichtungen 24 bis 26 ist so in dem Gehäuse bzw. an dem Element zur Signalerzeugung 20 positioniert, dass ihre Spulenachse in Richtung auf den Ort HWS gerichtet ist. Die anderen Spulen 22, 23, 27, 28 sind so angeordnet, dass Ihre Achsen auf den Ort LC gerichtet sind. Dies ist schematisch durch die entsprechenden Linien in Figur 3 angedeutet. Dies bedeutet insbesondere, dass die Spulenachsen z. B. auf den Ort LC oder HWS gerichtet sind, indem Teile 29, 30 des Signalerzeugungselements 20 gegenüber anderen Teilen 31, die jeweils Spuleneinrichtungen aufweisen, in der Richtung senkrecht zur Zeichenebene versetzt sind.

In der in Figur 3 dargestellten Ausführungsform sind die Spulen 22 bis 26 weiterhin so angeordnet, dass sie im Wesentlichen auf einem Halbkreisbogen liegen, der seinen Mittelpunkt im Behandlungsort hat. Dies bedeutet, dass sie alle gleich vom Behandlungsort LC beabstandet sind. Dies ist insbesondere dann vorteilhaft, wenn Spulen mit jeweils gleicher Feldstärke verwendet werden. Sollten die Spulen 22 bis 26 jedoch unterschiedliche Feldstärken aufweisen, so können sie selbstverständlich auch in unterschiedlichen Entfernungen vom Behandlungsort angeordnet werden, um insbesondere die Stärke des magnetischen Felds und dessen Ausrichtung am Behandlungsort LC entsprechend den jeweiligen Anforderungen einzustellen. Vorteilhafterweise sollte die Stärke des magnetischen Felds am Behandlungsort LC unter 20 Gauss liegen und die Frequenz mit der das Feld gepulst ist, sollte unter 30 Hz liegen. Dabei ist festzuhalten, dass die Spulen 22 bis 26 zum Erzielen eines guten Behandlungserfolgs mit beispielsweise Rechtecksimpulsen betrieben werden. Als günstig hat sich beispielsweise eine Entfernung der Spulen 22 bis 26 vom Behandlungsort LC von ca. 8 cm herausgestellt.

Der wesentliche Aspekt der Erfindung liegt darin, eine Magnetspule, insbesondere zur Anwendung in einem Gerät zur pulsierenden Signaltherapie, vorzusehen, die kostengünstig herzustellen ist und ein gut und genau gerichtetes Feld erzeugt, so dass eine gezielte Behandlung vorgenommen werden kann. Dies bedeutet insbesondere, dass die Streuung des Felds möglichst gering gehalten werden soll.

## Patentansprüche

1. Magnetspule (10), umfassend einen Kern (14) mit einer Längsachse und um den Kern gewickelte Spulenwindungen (12), wobei der Kern durch gewickeltes, dünnes Blech gebildet ist.

2. Magnetspule (10) nach Anspruch 1, **dadurch gekennzeichnet, dass** der Kern (14) im Wesentlichen kreiszylindrisch ist und das Blech um die Längsachse des Kreiszylinders gewickelt ist.

3. Magnetspule (10) nach einem der vorhergehenden Ansprüche, **dadurch gekennzeichnet, dass** die Spulenwindungen (12) aus Kupferdraht gebildet werden.

4. Magnetspule (10) nach einem der vorhergehenden Ansprüche, **dadurch gekennzeichnet, dass** der Kern (14) aus Fe-Blech gebildet wird.

5. Verwendung einer Magnetspule (10) nach einem der vorhergehenden Ansprüche in einem Gerät für die pulsierende Signaltherapie (20).

6. Verwendung nach Anspruch 5, wobei die Spule (22, 23, 24, 25, 26, 27, 28) so angeordnet ist, dass ihre Achse in der Einsatzposition des Geräts auf den Anwendungspunkt (LC) der Signaltherapie gerichtet ist.

7. Verwendung nach Anspruch 5 oder 6, **dadurch gekennzeichnet, dass** mehrere Spulen (22, 23, 24, 25, 26, 27, 28) vorgesehen sind.

8. Verwendung nach Anspruch 7, **dadurch gekennzeichnet, dass** die Spulen (22, 23, 24, 25, 26, 27, 28) auf einem fiktiven Kreis mit Mittelpunkt im Anwendungspunkt (LC) der Signaltherapie angeordnet sind und sich die Verlängerungen ihrer Achsen im Mittelpunkt dieses Kreises schneiden.
